# EUROPEAN PATENT SPECIFICATION

(11) **EP 1 122 235 B1**
(45) Date of publication and mention of the grant of the patent: **15.09.2004**
(21) Application number: 00303755.3
(22) Date of filing: 04.05.2000
(51) Int. Cl.: C07C 67/37, C07C 69/675, C07C 29/141, C07C 31/20

(54) **Process for preparing 1,3-alkanediol from epoxide derivative**
Verfahren zur Herstellung von 1,3-Alkandiol aus einem Epoxidderivat
Procédé de préparation d'alkane-1,3-diol d'un dérivé d'époxyde

(30) Priority: 03.02.2000 KR 2000005357
(43) Date of publication of application: 08.08.2001
(73) Proprietor: Davy Process Technology Limited, London W2 6LE (GB)
(72) Inventor: Lee, Byeong-No, Chungnang-gu, Seoul, 131-222 (KR); Chen, Byung-Soon, Yuseong-gu, Taejeon, 305-390 (KR)
(74) Representative: W.P. THOMPSON & CO.

(56) References cited:
- US-A- 4 410 744
- K. HINTERDING, ET AL.: "Regioselective carbomethoxylation of chiral epoxides: a new route to enantiomerically pure beta-hydroxy esters" JOURNAL OF ORGANIC CHEMISTRY, vol. 64, no. 7, 11 March 1999 (1999-03-11), pages 2164-2165, XP002216881 American Chemical Society, Washington, DC, US ISSN: 0022-3263

## Description

### Field of the Invention

The present invention relates to a process for preparing a 1,3-alkanediol through carbonylation of an epoxide derivative. More specifically, the present invention relates to a process for preparing 1,3-alkanediol, which comprises reacting an epoxide derivative with alcohol and carbon monoxide under the catalyst system consisting of a cobalt catalyst and a promoter to convert 3-hydroxyester, and adding hydrogen to the3-hydroxyester to prepare 1,3-alkanediol.

### Background of the Invention

An epoxide derivative can be easily converted into a difunctional compounds through carbonylation, which is used as an intermediate for preparing an organic compound. In particular, as 3-hydroxyester derivative has two functional groups, it is used as solvents, resins, coating materials, materials for medical substances, an intermediate for preparing alkanediol which is used for preparing polyester, etc. The alkanediol is used as an intermediate for coating materials or synthetic organic compounds. 1,3-diol is a representative of alkandiol. As shown in the following scheme, 1,3-diol is prepared by hydroformylating an epoxide derivative to prepare 3-hydroxyaldehyde derivative and adding hydrogen to the 3-hydroxyaldehyde derivative to convert the aldehyde groups into alcohol groups.

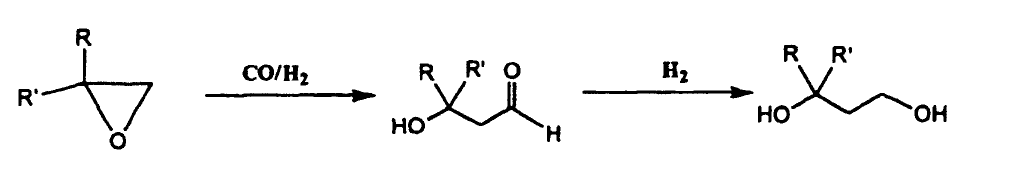

The scheme for preparing 1,3-diol above was disclosed in U.S. Patent Nos. 5,770,776, 5,723,389 and 5,731,478. On the other hand, in a known process for synthesizing 3-hydroxyaldehyde showing a high selectivity under a low temperature and a low pressure, there are used a cobalt catalyst and phosphine oxide ligand as a promoter. However, when phosphine oxide ligand is used as a promoter, the recovery and regeneration of the catalyst become complicated.

U.S. Patent No. 5,770,776 discloses a process for preparing 1,3-propanediol comprising the steps of (a) contacting ethylene oxide with carbon monoxide and hydrogen in a non-water-miscible solvent in the presence of an effective amount of a non-phosphine-ligated cobalt catalyst and an effective amount of a catalyst promoter. (b) adding an aqueous liquid to said intermediate product mixture and extracting into said aqueous liquid a major portion of the 3-hydroxypropanal so as to provide a first aqueous phase and a first organic phase, (c) separating the first aqueous phase from the first organic phase, (d) adding fresh non-water-miscible solvent to the first aqueous phase and extracting into such solvent a portion of any cobalt catalyst or cobalt-containing derivative thereof present in such aqueous phase, to provide asecond aqueous phase and a second organic phase, (e) separating the second aqueous phase from the second organic phase, (f) passing the first organic phase and the second organic phase to the process step (a), (g) contacting the second aqueous phase with hydrogen, and (h) recovering 1.3-propanediol.

U.S. Patent Nos. 5,723,389 and 5,731,478 disclose a process for preparing an alkanediol comprising the steps of (a) contacting an ethylene oxide with carbon monoxide and hydrogen in a non-water-miscible solvent in the presence of an effective amount of a non-phosphine-ligated cobalt or rhodium catalyst and an effective amount of a cobalt or rhodium porphyrin promoter, (b) adding an aqueous liquid to said intermediate product mixture and extracting into said aqueous liquid a major portion of the hydroxyaldehyde so as to provide an aqueous phase and an organic phase, (c) separating the aqueous phase from the organic phase, (d) contacting the aqueous phase with hydrogen in the presence of a hydrogenation catalyst, and (e) recovering the alkanediol.

U.S. Patent Nos. 5,135,901 and 4,973,741 disclose another process for obtaining the 3-hydroxyester derivative from the epoxide derivatives. In this process, there is synthesized methyl 3-hydroxypropionate from ethylene oxide by using rhodium and ruthenium as catalysts in the presence of carbon monoxide and alcohol. However, in this process, in spite of the use of expensive catalysts, the yield of the 3-hydroxypropionate is as low as 60%, and by-products are produced in considerable amounts. Further, there is another known process for obtaining a 3-hydroxyester by hydroesterification of the epoxide. In this process also, the yield is as low as 40-60% [(1) Dalcanali, E.; Foa, M. Synthesis 1986, 492. (2) Heck, R. F., J. Am. Chem. Soc., 1963, 85, 1460. (3) Eismann, J. L.; Yamartino, R. L.; Howard, Jr. J. F., J. Org. Chem. 1961, 2102.]. The reason why the yield is so low is that the isomerization reaction of the starting material readily occurs.

Meanwhile, U.S. Patent Nos. 5,310,948 and 5,359,081 relate to a carbonylation of the epoxide, in which the epoxide and carbon monoxide are reacted in the presence of cobalt and pyridine derivatives. The final product is mainly *β*-lacton, and the by-product is the 3-hydroxyester.

In preparation of 1,3-alkanediol for preparing polyester, if 3-hydroxyaldehyde is used as intermediate, the quality of polyester becomes lower because of formation of oligomers and acetals as by-products. On the other hand, if 3-hydroxyester is used as intermediate, a lower yield and a high cost of catalyst will arise.

An effective catalyst system for preparing a 3-hydroxyester derivative from an ethylene oxide through hydroesterification has not been developed yet, and a method of obtaining 1,3-alkanediol in a high yield has not been developed yet.

Accordingly, the present inventors have developed a process for preparing 1,3-alkanediol in a high yield by synthesizing 3-hydroxyester as an intermediate from an epoxide derivative through hydroesterification and reacting the 3-hydroxyester with hydrogen and a catalyst system for the hydroesterification.

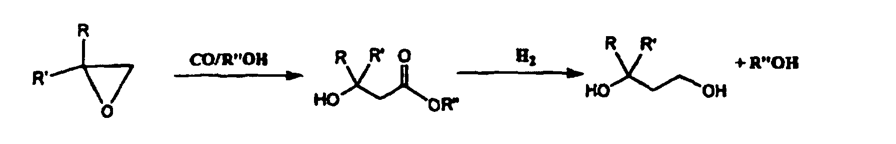

### Objects of the Invention

An object of the present invention is to provide a novel process for preparing 1,3-alkanediol, which comprises reacting an epoxide derivative with alcohol and carbon monoxide under the catalyst system consisting of a cobalt catalyst and a promoter to convert 3-hydroxyester, and adding hydrogen to the3-hydroxyester to prepare 1,3-alkanediol.

Another object of the invention is to provide a new catalyst system for preparing 3-hydroxyester in a high yield by reacting an epoxide derivative with alcohol and carbon monoxide, which consists of a cobalt catalyst and an imidazole or a derivative thereof as promoter.

A further object of the invention is to provide a process for preparing 1,3-alkanediol using a new catalyst system for preparing 3-hydroxyester in a high yield by reacting an epoxide derivative with alcohol and carbon monoxide, which consists of a cobalt catalyst and an imidazole or a derivative thereof as promoter.

A further object of the invention is to provide a process for preparing 1,3-alkanediol in a lower cost by using an imidazole or a derivative thereof as promoter.

In achieving the above objects, the present invention is characterized as follows.

### Summary of the Invention

The present invention relates to a process for preparing 1,3-alkanediol through carbonylation of an epoxide derivative, which comprises (a) reacting an epoxide derivative with alcohol and carbon monoxide in a solvent at a temperature of from 30 to 150 °C and at a pressure of from 50 to 3000 psig (446 to 20786 kPa (absolute)) under the catalyst system consisting of an effective amount of a cobalt catalyst and an effective amount of a promoter selected from imidazole, pyrrole, pyrazine, pyrimidine, derivatives thereof and mixtures thereof to convert 3-hydroxyester and derivatives thereof in an amount of from 2 to 95% by weight, (b) separating said reaction product and solvent from the catalyst and promoter, (c) reacting said reaction product and solvent with hydrogen at a temperature of from 30 to 350 °C and at a pressure of from 50 to 5000 psig (446 to 34576 kPa (absolute)) under a catalyst system for hydrogenation to prepare a hydrogenation product mixture including 1,3-alkandiol, and (d) recovering 1,3-alkandiol from the hydrogenation product mixture.

### Detailed Description of the Invention

The present invention relates to a process for preparing 1,3-alkanediol through carbonylation of an epoxide derivative. An epoxide derivative reacts with alcohol and carbon monoxide in a solvent at a temperature of from 30 to 150 °C and at a pressure of from 50 to 3000 psig (446 to 20786 kPa (absolute)) under the catalyst system consisting of an effective amount of a cobalt catalyst and an effective amount of a promoter selected from imidazole, pyrrole, pyrazine, pyrimidine, derivatives thereof and mixtures thereof to convert 3-hydroxyester and derivatives thereof. In this invention, this step is called as "hydroesterification". The reaction product and solvent is separated from the catalyst and promoter. The reaction product and solvent reacts with hydrogen at a temperature of from 30 to 350 C and at a pressure of from 50 to 5000 psig (446 to 34576 kPa (absolute)) under a catalyst system for hydrogenation to prepare a hydrogenation product mixture including 1,3-alkandiol. In this invention, this step is called as "hydrogenation". 1,3-allcandiol is recovered from the hydrogenation product mixture.

In the present invention, an effective catalyst system is employed to maximize the production yield of 3-hydroxyester when an epoxide derivative carries out hydroesterification. The catalyst system consists of a cobalt catalyst and a catalyst promoter. A representative example of the cobalt catalyst is Co₂(CO)₈. The cobalt catalyst may be a compound of Co₂(CO)₈ and an organic compound selected from the group consisting of imidazole, pyrrole, pyrazine, pyrimidine, a derivative thereof, and a mixture thereof. It is preferable to use an organic compound which is not bonded to a phosphine compound.

It is preferable to use the cobalt catalyst and promoter at a ratio of up to 1 : 100. In the present invention, it is also preferable to use an imidazole derivative as a promoter as shown in the following formula (I): 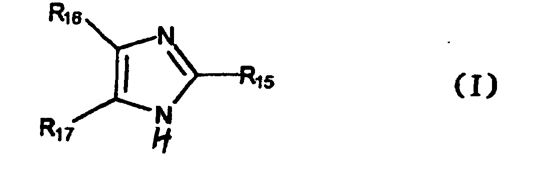 wherein R₁₅, R₁₆, and R₁₇ are independently hydrogen; branched aliphatic hydrocarbon having up to 10 carbon atoms; linear aliphatic hydrocarbon having up to 10 carbon atoms; saturated cyclohydrocarbon having up to 10 carbon atoms; cycloaliphatic hydrocarbon having up to 10 carbon atoms; aromatic aliphatic hydrocarbon having up to 10 carbon atoms; F; Cl; alkoxy of C₁₋₃; OH; OH group-containing branched aliphatic hydrocarbon having up to 10 carbon atoms; OH group-containing linear aliphatic hydrocarbon having up to 10 carbon atoms; OH group-containing saturated cyclohydrocarbon having up to 10 carbon atoms; OH group-containing cycloaliphatic hydrocarbon having up to 10 carbon atoms; or OH group-containing aromatic aliphatic hydrocarbon having up to 10 carbon atoms.

An appropriate solvent is used in the hydroesterification in the presence of alcohol. The hydroesterification is carried out at a temperature of from 30 to 150 °C, preferably from 40 to 120 °C and at the CO pressure of from 50 to 3000 psig (446 to 34576 kPa (absolute). preferably from 100 to 1500 psig (790 to 10444 kPa (absolute))

The epoxide derivative usable in this invention is shown as the following formula (II): 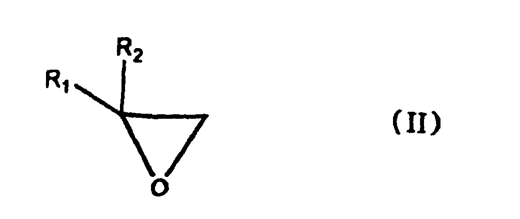 wherein R, and R, are independently hydrogen; linear aliphatic hydrocarbon having up to 20 carbon atoms; branched aliphatic hydrocarbon having up to 20 carbon atoms; saturated cycloaliphatic hydrocarbon having up to 20 carbon atoms; cycloaliphatic hydrocarbon having up to 20 carbon atoms; aromatic aliphatic hydrocarbon having up to 20 carbon atoms; hydrocarbon having up to 20 carbon atoms substituted hydrogen on the carbon chain for F or Cl or Br; aromatic hydrocarbon having up to 20 carbon atoms with no substituted group or aromatic hydrocarbon having up to 20 carbon atoms substituted hydrogen on the aromatic ring for F, Cl, amine group, nitrile group, or alkoxy group.

The desirable examples of the epoxide derivatives include ethylene oxide, propylene oxide, 1-butene oxide, 1-pentene oxide, 1-heptene oxide, 1-octene oxide, 1-nonene oxide, 1-decene oxide, 2-methyl-propylene oxide, 2-methyl-1-butene oxide, 2-methyl-1-octene oxide, 2-methyl-nonene oxide, 2-methyl-1-decene oxide, 2-methyl-1-butene oxide, 2-methyl-1-pentene oxide, 2-methyl-1-hexene oxide, 2-ethyl-1-heptene oxide, 2-ethyl-1-octene oxide, 2-ethyl-1-nonene oxide, 2-ethyl-1-decene oxide, epifluorohydrin, epichlrorohydrin, epibromohydrin, glycidol, methyl gltcidate, ethyl glycidate, t-butyl glycidate, allyl benzene oxide, styrene oxide, etc.

The alcohol of the present invention is expressed by R'OH. Here, R' is a a saturated linear hydrocarbon having up to 20 carbon atoms, an unsaturated linear hydrocarbon having up to 20 carbon atoms; a hydrocarbon having up to 20 carbon atoms having branches, a cyclohydrocarbon having up to 20 carbon atoms, an aromatic hydrocarbon having up to 20 carbon atoms, or a linear hydrocarbon including an aromatic group.

Preferably R' is methyl, ethyl, isopropyl, cyclohexyl, phenyl or benzyl.

The solvent of the present invention may be an ether, a substituted aromatic compound, or an acetate. Of course, the alcohol itself may be used as a solvent.

The ether available in this invention is represented as the following formula (III), (IV) or (V):

**R**_{**3**}**―O―R**_{**4**} (III)

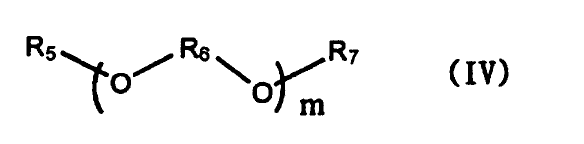

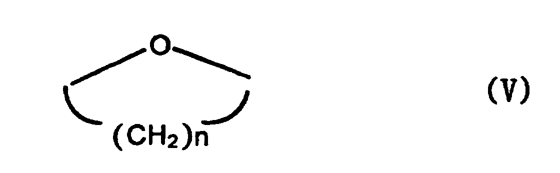

wherein R₃, R₄, R₅, R₆, and R₇ are saturated aliphatic hydrocarbon having up to 10 carbon atoms; having no branches; branched aliphatic hydrocarbon having up to 10 carbon atoms; saturated cyclohydrocarbon having up to 10 carbon atoms; cycloaliphatic hydrocarbon having up to 10 carbon atoms; or aromatic aliphatic hydrocarbon having up to 10 carbon atoms; m is an integer of 1 to 10; and n is an integer of 2 to 5.

The substituted aromatic compound available in this invention is represented as the following formula (VI): 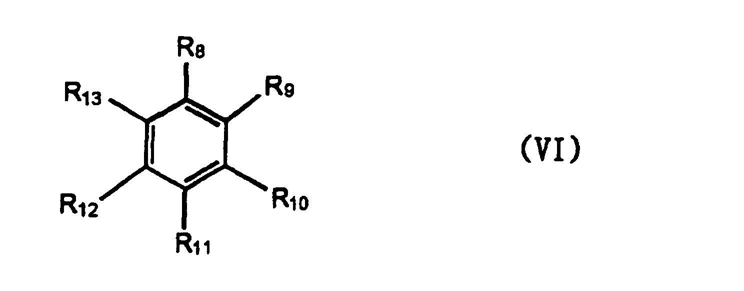 where R₈, R₉, R₁₀, R₁₁, R₁₂ and R₁₃ are hydrogen; saturated aliphatic hydrocarbon of C₁₋₄ having no branches; branched aliphatic hydrocarbon of C₁₋₄; F; Cl; or alkoxy group of C_{1∼3}.

3-hydroxyester and derivatives thereof are obtained through hydroesterification of the epoxide derivative. The 3-hydroxyester and derivatives thereof according to the present invention are represented by the following formula (VII) or (VIII): 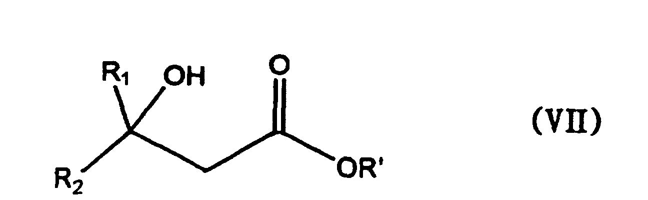 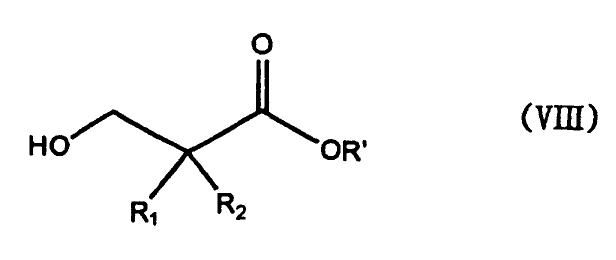 wherein R₁ and R₂ are hydrogen; linear aliphatic hydrocarbon having up to 20 carbon atoms; branched aliphatic hydrocarbon having up to 20 carbon atoms; saturated cycloaliphatic hydrocarbon having up to 20 carbon atoms; cycloaliphatic hydrocarbon having up to 20 carbon atoms; aromatic aliphatic hydrocarbon having up to 20 carbon atoms; hydrocarbon having up to 20 carbon atoms substituted hydrogen on the carbon chain for F or Cl or Br; aromatic hydrocarbon having up to 20 carbon atoms with no substituted group; or aromatic hydrocarbon having up to 20 carbon atoms substituted hydrogen on the aromatic ring for F, Cl, amine group, nitrile group, or alkoxy group, and R' is a saturated linear hydrocarbon having up to 20 carbon atoms, an unsaturated linear hydrocarbon having up to 20 carbon atoms, a hydrocarbon having up to 20 carbon atoms having branches, a cyclohydrocarbon having up to 20 carbon atoms, an aromatic hydrocarbon of having up to 20 carbon atoms, or a linear hydrocarbon including an aromatic group.

As shown in formulae (VII) and (VIII), the 3-hydroxyester and derivatives thereof are difunctional so as to be available for an intermediate for synthesizing an organic compound or for a coating material.

The reaction product and solvent is separated from the catalyst and promoter. The separation of the reaction product and solvent is carried out by vacuum distillation or by extracting into water, which will depend on the solvent. When an ether of Formula (III) or a substituted aromatic compound of Formula (IV) is used as solvent, the reaction product of 3-hydroxyester derivative is extracted into water. When an ether of Formula (IV) or (V) or a lower alcohol such as methyl alcohol, ethyl alcohol and isopropyl alcohol is used as solvent, the reaction product is separated under vacuum distillation. When a higher alcohol than isopropyl alcohol is used as solvent, the reaction product is separated by extracting into water. When extracting into water, 3-hydroxyester and derivatives thereof are extracted into the water layer by adding water of 100 °C or lower to the reaction production mixture in the presence of carbon monoxide at a pressure of from 20 to 3000 psig (239 to 20786 kPa (absolute).

The extracts react with hydrogen at a temperature of from 30 to 350 °C and at a pressure of from 50 to 5000 psig (446 to 34576 kPa (absolute)) under a catalyst system for hydrogenation to prepare a hydrogenation product mixture including 1,3-alkandiol. In this invention, this step is called as "hydrogenation". The hydrogenation can be carried out without extraction of the reaction product. The separated catalyst and promoter can be recycled to the hydroesterification in partial or in total. The 3-hydroxyester derivative is hydrogenated under a catalyst system to produce 1,3-alkanediol. The catalyst system comprises copper chromate or Pd/C. The hydrogenation process is carried out at the temperature of from 50 to 250 °C and at the pressure of from 200 to 3000 psig (1480 to 20786 kPa (absolute)). Finally, 1,3-alkandiol is recovered from the hydrogenation product mixture. The separation and hydrogenation of the reaction product and the hydrogenation can be easily carried out by an ordinary skilled person in the art to which the present invention pertains.

The present invention will be more thoroughly understood by the below described actual examples. These examples are not intended to limit the scope of the present invention, but are just for specifically presenting the present invention to help understanding the present invention.

### Examples

### Examples 1-10: Hydroesterification of Ethylene Oxide with Catalyst of Co₂(CO)₈ in Imidazole

At room temperature, a 450 ml Parr reactor was filled with a solvent and 5 mmole of Co₂(CO)₈. The reactor was filled with CO gas at 500 psig (3549 kPa (absolute)), heated to 80 °C and agitated for one hour. Then the reactor was cooled to room temperature and imidazole as promoter was added to the reactor. Ethylene oxide was added to the reactor and CO was added at a predetermined pressure. The reactor was heated to a temperature as shown in Table 1 at a pressure as in Table 1 for the time as in Table 1. During reaction, the reaction product was sampled using a tube. As product, methyl 3-hydroxypropionate (3-HPM) was analyzed with a GC. The reaction conditions and the resulting data are shown in Table 1.

Imidazole of 20 mmole as promoter was used in Examples 1-7 and 9, 40 mmole in Example 8, and 30 mmole in Example 10. Ethylene oxide of 500 mmole was used in Examples 1-8 and 10, and 1.4 mmole in Example 9. In Example 7, tetraglyme was used besides methyl alcohol.

**Table 1**

| Example | Temp. (°C) | Pressure (bar) (kPa) | Time (hrs) | MeOH (ml) | Conversion Rate(%) | Yield¹⁾(%) | Selectivity(mole%) | | | | |
|---|---|---|---|---|---|---|---|---|---|---|---|
| | | | | | | 3-HPM | 3-HPM²⁾ | AA³⁾ | DMA⁴⁾ | ME⁵⁾ | Dimer⁶⁾ |
| 1 | 70 | 34 (3400) | 3 | 200 | 78.09 | 65.66 | 84.08 | 2.71 | 9.44 | 1.10 | 2.66 |
| 2 | 70 | 50 (5000) | 3 | 200 | 68.23 | 59.68 | 87.47 | 7.77 | 0.20 | 2.38 | 2.17 |
| 3 | 70 | 80 (8000) | 3 | 200 | 65.66 | 58.65 | 89.33 | 4.50 | 0.59 | 2.47 | 3.11 |
| 4 | 60 | 50 (5000) | 3 | 200 | 45.19 | 40.20 | 88.96 | 8.45 | 0.39 | 2.19 | 0 |
| 5 | 75 | 50 (5000) | 3 | 200 | 78.62 | 66.01 | 83.97 | 0.03 | 10.98 | 1.92 | 3.10 |
| 6 | 80 | 50 (5000) | 3 | 200 | 91.61 | 71.86 | 78.45 | 9.44 | 6.51 | 2.63 | 2.97 |
| 7 | 80 | 34 (3400) | 2 | 100 | - | 82.44 | - | - | - | - | - |
| 8 | 80 | 34 (3400) | 4 | 250 | - | 70.1 | - | - | - | - | - |
| 9 | 80 | 34 (3400) | 4 | 200 | - | 66.4 | - | - | - | - | - |
| 10 | 75 | 60 (6000) | 3 | 150 | 95.27 | 88.90 | 93.31 | 1.78 | 0.32 | 0.98 | 3.61 |

| | | | | | | | | | | | |
|---|---|---|---|---|---|---|---|---|---|---|---|
| Notes: 1) Yield = Selectivity X Conversion rate | | | | | | | | | | | |
| 2) 3-HPM: methyl 3-hydroxypropionate or 3hydroxypropionic acid methyl ester | | | | | | | | | | | |
| 3) AA: acetaldehyde | | | | | | | | | | | |
| 4) DMA: acetaldehyde dimethyl acetal | | | | | | | | | | | |
| 5) ME: methoxy ethanol | | | | | | | | | | | |
| 6) Dimer: HOCH₂CH₂C(O)OCH₂CH₂(O)OCH₃ | | | | | | | | | | | |

### Comparative Examples 1-2: Hydroesterification of Ethylene Oxide with Catalyst of Co₂(CO)₈

Comparative Example 1 was conducted in the same manner as in Example 1 except that 4 mmole of 3-hydroxypyridine was used as promoter instead of imidazole and except the reaction conditions. 1 mmole of Co₂(CO)₈ was used and 200 mmole of ethylene oxide was used. The reaction conditions of Comparative Example 1 were shown in Table 2.

Comparative Example 2 was conducted in the same manner as in Example 1 except that Co₂(CO)₈ as catalyst was used without any catalyst promoter. 2.5 mmole of Co₂(CO)₈ was used and 650 mmole of ethylene oxide was used.The reaction conditions of Comparative Example 2 were shown in Table 2. the resulting data were shown in Table 2.

In Comparative Example 1 using a pyridine derivative, 3-HPM was obtained in a high yield, but by-products such as acetaldehyde was produced in a significant amount. In Comparative Example 2 not using a catalyst promoter, the yield of 3-HPM was very low.

**Table 2**

| Comparative Example | Temp (°C) | Pressure (bar) (kPa) | Time (hrs) | MeOH (ml) | Conversion Rate(%) | Yield¹⁾(%) | Selectivity(mole%) | | | | |
|---|---|---|---|---|---|---|---|---|---|---|---|
| | | | | | | 3-HPM | 3-HPM²⁾ | AA³⁾ | DMA⁴⁾ | ME⁵⁾ | Dimer⁶⁾ |
| 1 | 75 | 60 (6000) | 4 | 40 | 92.07 | 81.08 | 88.07 | 8.43 | - | 2.28 | 1.22 |
| 2 | 75 | 60 (6000) | 4 | 120 | - | 20.6 | - | - | - | - | - |

| | | | | | | | | | | | |
|---|---|---|---|---|---|---|---|---|---|---|---|
| Notes: 1) Yield = Selectivity X Conversion rate | | | | | | | | | | | |
| 2) 3-HPM: methyl 3-hydroxypropionate or 3hydroxypropionic acid methyl ester | | | | | | | | | | | |
| 3) AA: acetaldehyde | | | | | | | | | | | |
| 4) DMA: acetaldehyde dimethyl acetal | | | | | | | | | | | |
| 5) ME: methoxy ethanol | | | | | | | | | | | |
| 6) Dimer: HOCH₂CH₂C(O)OCH₂CH₂(O)OCH₃ | | | | | | | | | | | |

### Examples 11-14

Examples 11-14 were conducted in the same manner as in Example 1 except the epoxide derivative. As shown in Table 3, in Examples 11-14, propylcne oxide, butylenes oxide, epichlorohydrin, and glycidol as epoxide derivative were used, respectively. 5 mmole of Co₂(CO)₈, 10 mmole of catalyst promoter, 500 mmole of epoxide derivative, and 200 ml of ethyl alcohol as solvent was used. The reactor was kept at 80 °C and at 34 bar (3400 kPa) for 4 hours. The products and yields of Examples 11-14 were shown in Table 3.

**Table 3**

| Example | Epoxide | Product | Yield(%) |
|---|---|---|---|
| 1 | propylene oxide- | methyl 3-hydroxybutanoate | 60.56 |
| 2 | butylene oxide | methyl 3-hydroxypentanoate | 53.70 |
| 3 | epichlorohydrin | methyl 3-hydroxy4-chlorobutanoate | 66.17 |
| 4 | glycidol | 3-hydroxy *γ*-butyrolacton | 62.50 |

### Example 15

To a 45 ml Parr reactor were 1 g of 3-hydroxy propionate and 10 ml of methyl alcohol added to dissolve the 3-hydroxy propionate. To the reactor was 0.5 g of copper chromate as catalyst added. Hydrogen was introduced to the reactor at 1500 psig (34576 kPa (absolute) and the reactor was agitated and heated to 180 °C. After reaction for 15 hours, the reactor was cooled to room temperature. The product was analyzed with a GC. Conversion rate to 3-hydroxy propionate was about 5 %, and selectivity to 1,3-propandiol was about 3 %.

The present invention has an effect to provide a novel process for preparing 1,3-alkanediol, in which 3-hydroxyester is prepared in a high yield by reacting an epoxide derivative with alcohol and carbon monoxide under a catalyst system consisting of a cobalt catalyst and an imidazole or a derivative thereof as promoter. Further, the present invention has an effect to provide a process for preparing 1,3-alkanediol in a lower cost by using an imidazole or a derivative thereof as promoter.

## Claims

1. A process for preparing 1,3-alkanediol through carbonylation of an epoxide derivative, which comprises:
(a) reacting an expoxide derivative with alcohol and carbon monoxide in a solvent at a temperature of from 30 to 150 °C and at a pressure of from 50 to 3000 psig (446 to 20786 kPa(absolute)) under the catalyst system consisting of an effective amount of a cobalt catalyst and an effective amount of promoter selected from imidazole, pyrrole, pyrazine, pyrimidine, derivates thereof and mixtures thereof to convert 3-hydroxyester and derivatives thereof;
(b) separating said reaction product and solvent from the catalyst and promoter;
(c) reacting said reaction product and solvent with hydrogen at a temperature of from 30 to 350 °C and at a pressure of from 50 to 5000 psig (446 to 34576 kPa(absolute)) under a catalyst system for hydrogenation to prepare a hydrogenation product mixture including 1,3-alkandiol; and
(d) recovering 1,3-alkandiol from the hydrogenation product mixture.

2. The process as claimed in Claim 1, wherein said separating step (b) is conducted under vacuum distillation.

3. The process as claimed in Claim 1, wherein said separating step (b) is conducted by extracting 3-hydroxyester and derivatives thereof into a water layer by adding water at a temperature of 100°C or lower to the reaction production mixture in the presence of carbon monoxide at a pressure of from 20 to 3000 psig (239 to 20786 kPa(absolute)).

4. The process as claimed in any of Claims 1 to 3, wherein said catalyst and promoter are recycled to the step (a) for hydroesterification in partial or in total.

5. The process as claimed in any of Claims 1 to 4, wherein said cobalt catalyst is Co₂(CO)₈.

6. The process as claimed in any of Claims 1 to 5, wherein said cobalt catalyst and promoter are employed at a ratio of up to 1:100.

7. The process as claimed in any of Claims 1 to 6, wherein said promoter is an imidazole derivative represented by the following formula (I): 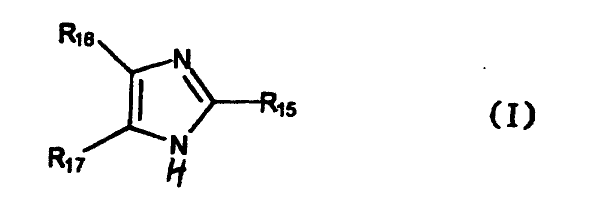 wherein R₁₅, R₁₆, and R₁₇ are independently hydrogen; branched aliphatic hydrocarbon having up to 10 carbon atoms; linear aliphatic hydrocarbon having up to 10 carbon atoms; saturated cyclohydrocarbon having up to 10 carbon atoms; cycloaliphatic hydrocarbon having up to 10 carbon atoms; aromatic aliphatic hydrocarbon having up to 10 carbon atoms; F; Cl; alkoxy of C₁₋₃; OH; OH group-containing branched aliphatic hydrocarbon having up to 10 carbon atoms; OH group-containing linear aliphatic hydrocarbon having up to 10 carbon atoms; OH group-containing saturated cyclohydrocarbon having up to 10 carbon atoms; OH group-containing cycloaliphatic hydrocarbon having up to 10 carbon atoms; or OH group-containing aromatic aliphatic hydrocarbon having up to 10 carbon atoms.

8. The process as claimed in Claim 7, wherein said promoter is an imidazole.

9. The process as claimed in any of Claims 1 to 8, wherein said reacting step (a) is conducted at a temperature of from 40 to 120°C.

10. The process as claimed in any of claims 1 to 9, wherein said reacting step (a) is conducted at a pressure of from 100 to 1500 psig (790 to 10444 kPa(absolute)).

11. The process as claimed in any of Claims 1 to 10, wherein said epoxide derivative is represented by the following formula (II): 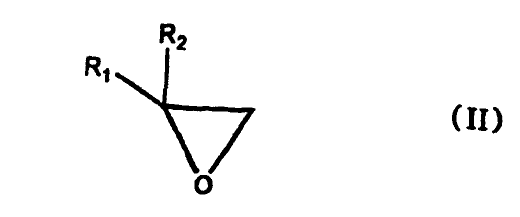 wherein R₁ and R₂ are independently hydrogen; linear aliphatic hydrocarbon having up to 20 carbon atoms; branched aliphatic hydrocarbon having up to 20 carbon atoms; saturated cycloaliphatic hydrocarbon having up to 20 carbon atoms; cycloaliphatic hydrocarbon having up to 20 carbon atoms; aromatic aliphatic hydrocarbon having up to 20 carbon atoms; hydrocarbon having up to 20 carbon atoms substituted hydrogen on the carbon chain for F or Cl or Br; aromatic hydrocarbon having up to 20 carbon atoms with no substituted group or aromatic hydrocarbon having up to 20 carbon atoms substituted hydrogen on the aromatic ring for F, Cl, amine group, nitrile group, or alkoxy group.

12. The process as claimed in any of Claims 1 to 11, wherein said alcohol is represented by R'OH, wherein R' is a saturated linear hydrocarbon having up to 20 carbon atoms, an unsaturated linear hydrocarbon having up to 20 carbon atoms; a hydrocarbon having up to 20 carbon atoms having branches, a cyclohydrocarbon having up to 20 carbon atoms, an aromatic hydrocarbon having up to 20 carbon atoms, or a linear hydrocarbon including an aromatic group.

13. The process as claimed in any of Claims 1 to 12, wherein said solvent is represented by the following formula (III), (IV) or V):
**R**_{**3**}**―O―R**_{**4**} (III) 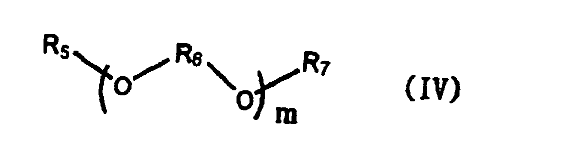 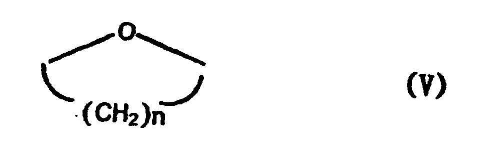
wherein R₃, R₄, R₅, R₆, and R₇ are saturated aliphatic hydrocarbon having up to 10 carbon atoms; having no branches; branched aliphatic hydrocarbon having up to 10 carbon atoms; saturated cyclohydrocarbon having up to 10 carbon atoms; cycloaliphatic hydrocarbon having up to 10 carbon atoms; or aromatic aliphatic hydrocarbon having up to 10 carbon atoms; m is an integer of 1 to 10; and n is an integer of 2 to 5.

14. The process as claimed in any of claims 1 to 12, wherein said solvent is an acetate.

15. The process as claimed in any of Claims 1 to 12, wherein said solvent is an alcohol represented by R'OH, wherein R' is a saturated linear hydrocarbon having up to 20 carbon atoms, an unsaturated linear hydrocarbon having up to 20 carbon atoms, a hydrocarbon having up to 20 carbon atoms having branches, a cyclohydrocarbon having up to 20 carbon atoms, an aromatic hydrocarbon having up to 20 carbon atoms or a linear hydrocarbon including an aromatic group.

16. The process as claimed in any of Claims 1 to 12, wherein said solvent is a substituted aromatic compound represented by the following formula (VI): 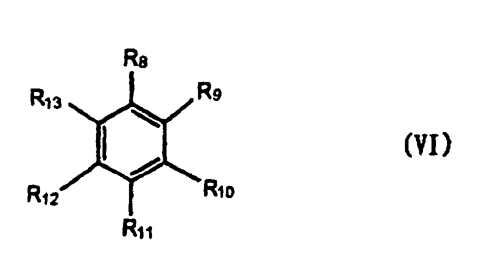 where R₈, R₉, R₁₀, R₁₁, R₁₂ and R₁₃ are hydrogen; saturated aliphatic hydrocarbon of C₁₋₄ having no branches; branched aliphatic hydrocarbon of C₁₋₄; F; Cl; or alkoxy group of C₁₋₃.

17. The process as claimed in any of Claims 1 to 16, wherein said reaction product includes 3-hydroxyester and derivatives thereof represented by the following formula (VII) or (VIII): 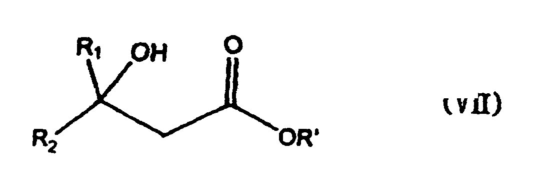 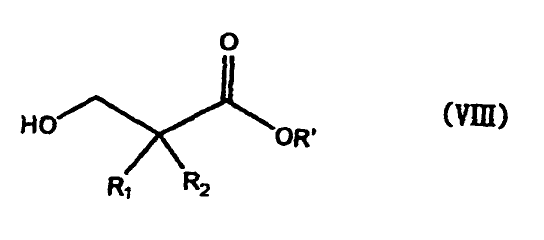 wherein R₁ and R₂ are hydrogen; linear aliphatic hydrocarbon having up to 20 carbon atoms; branched aliphatic hydrocarbon having up to 20 carbon atoms; saturated cycloaliphatic hydrocarbon having up to 20 carbon atoms; cycloaliphatic hydrocarbon having up to 20 carbon atoms; aromatic aliphatic hydrocarbon having up to 20 carbon atoms; hydrocarbon having up to 20 carbon atoms substituted hydrogen on the carbon chain for F or Cl or Br; aromatic hydrocarbon having up to 20 carbon atoms with no substituted group; or aromatic hydrocarbon having up to 20 carbon atoms substituted hydrogen on the aromatic ring for F, Cl, amine group, nitrile group, or alkoxy group, and R' is a saturated linear hydrocarbon having up to 20 carbon atoms, an unsaturated linear hydrocarbon having up to 20 carbon atoms, a hydrocarbon having up to 20 carbon atoms having branches, a cyclohydrocarbon having up to 20 carbon atoms, an aromatic hydrocarbon of having up to 20 carbon atoms, or a linear hydrocarbon including an aromatic group.

18. The process as claimed in any of claims 1 to 17, wherein said catalyst system of said step (c) is copper chromate or Pd/C.

19. The process as claimed in any of claims 1 to 18, wherein said step (c) is conducted at the pressure of hydrogen of from 200 to 3000 psig (1480 to 20786 kPa(absolute)).

20. The process as claimed in any of Claims 1 to 19, wherein said step (c) is conducted at the temperature of from 50 to 250°C.

## Patentansprüche

1. Verfahren zur Herstellung von 1,3-Alkandiol durch Carbonylierung eines Epoxid-Derivats, welches umfasst:
(a) Reagierenlassen eines Epoxid-Derivats mit Alkohol und Kohlenmonoxid in einem Lösungsmittel bei einer Temperatur von 30 bis 150°C und bei einem Druck von 50 bis 3000psig (446 bis 20786 kPa absolut) unter einem Katalysatorsystem, das aus einer wirksamen Menge eines Kobalt-Katalysators und einer wirksamen Menge eines Förderers besteht, der ausgewählt ist aus Imidazol, Pyrrol, Pyrazin, Pyrimidin, deren Derivaten und deren Mischungen, um 3-Hydroxyester und deren Derivate umzuwandeln;
(b) Trennen des Reaktionsprodukts und des Lösungsmittels vom Katalysator und dem Förderer;
(c) Reagierenlassen des Reaktionsprodukts und des Lösungsmittels mit Wasserstoff bei einer Temperatur von 30 bis 350°C und bei einem Druck von 50 bis 5000 psig (446 bis 34576 kPa absolut) unter einem Katalysatorsystem zur Hydrogenierung, um eine 1,3-Alkandiol enthaltende Hydrogenierungsproduktmischung herzustellen;
(d) Wiedergewinnen von 1,3-Alkandiol aus der Hydrogenierungsproduktmischung.

2. Verfahren gemäß Anspruch 1, wobei der Trennungsschritt (b) unter Vakuumdestillation durchgeführt wird.

3. Verfahren gemäß Anspruch 1, wobei der Trennungsschritt (b) durch Extrahieren von 3-Hydroxyestern und deren Derivaten in eine Wasserschicht durch Hinzugeben von Wasser, mit einer Temperatur von 100°C oder niedriger, zur Reaktionsproduktmischung in Anwesenheit von Kohlenmonoxid bei einem Druck von 20 bis 3000 psig (239 bis 20786 kPa absolut) durchgeführt wird.

4. Verfahren gemäß einem der Ansprüche 1 bis 3, wobei der Katalysator und der Förderer zum Schritt (a) zur teilweisen oder vollständigen Hydroveresterung zurückgeführt werden.

5. Verfahren gemäß einem der Ansprüche 1 bis 4, wobei der Kobalt-Katalysator Co₂(CO)₈ ist.

6. Verfahren gemäß einem der Ansprüche 1 bis 5, wobei der Kobalt-Katalysator und der Förderer in einem Verhältnis von bis zu 1:100 eingesetzt werden.

7. Verfahren gemäß einem der Ansprüche 1 bis 6, wobei der Förderer ein Imidazol-Derivat ist, das repräsentiert wird durch die folgende Formel (I): 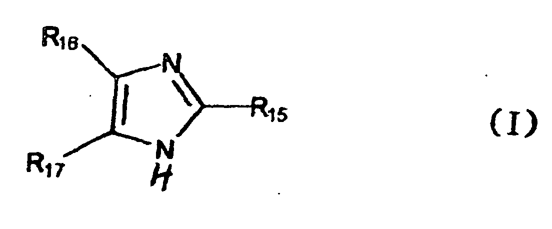 wobei R₁₅, R₁₆ und R₁₇ unabhängig voneinander Wasserstoff, verzweigter aliphatischer Kohlenwasserstoff mit bis zu 10 Kohlenstoffatomen; linearer aliphatischer Kohlenwasserstoff mit bis zu 10 Kohlenstoffatomen; gesättigter zyklischer Kohlenwasserstoff mit bis zu 10 Kohlenstoffatomen; cykloaliphatischer Kohlenwasserstoff mit bis zu 10 Kohlenstoffatomen; aromatischer aliphatischer Kohlenwasserstoff mit bis zu 10 Kohlenstoffatomen; F; Cl; C₁₋₃-Alkoxy; OH; OH-Gruppen enthalternder verzweigter aliphatischer Kohlenwasserstoff mit bis zu 10 Kohlenstoffatomen; OH-Gruppen enthalternder linearer aliphatischer Kohlenwasserstoff mit bis zu 10 Kohlenstoffatomen; OH-Gruppen enthalternder gesättigter zyklischer Kohlenwasserstoff mit bis zu 10 Kohlenstoffatomen; OH-Gruppen enthalternder cykloaliphatischer Kohlenwasserstoff mit bis zu 10 Kohlenstoffatomen; oder OH-Gruppen enthalternder aromatischer aliphatischer Kohlenwasserstoff mit bis zu 10 Kohlenstoffatomen sind.

8. Verfahren gemäß Anspruch 7, wobei der Förderer ein Imidazol ist.

9. Verfahren gemäß einem der Ansprüche 1 bis 8, wobei der Reaktionsschritt (a) bei einer Temperatur von 40 bis 120°C durchgeführt wird.

10. Verfahren gemäß einem der Ansprüche 1 bis 9, wobei der Reaktionsschritt (a) bei einem Druck von 100 bis 1500 psig (790 bis 10444 kPa absolut) durchgeführt wird.

11. Verfahren gemäß einem der Ansprüche 1 bis 10, wobei das Epoxid-Derivat repräsentiert wird durch die folgende Formel (II): 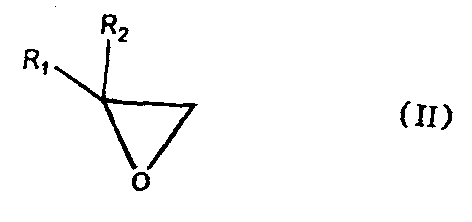 wobei R₁ und R₂ unabhängig voneinander Wasserstoff, linearer aliphatischer Kohlenwasserstoff mit bis zu 20 Kohlenstoffatomen; verzweigter aliphatischer Kohlenwasserstoff mit bis zu 20 Kohlenstoffatomen; gesättigter cykloaliphatischer Kohlenwasserstoff mit bis zu 20 Kohlenstoffatomen; cykloaliphatischer Kohlenwasserstoff mit bis zu 20 Kohlenstoffatomen; aromatischer aliphatischer Kohlenwasserstoff mit bis zu 20 Kohlenstoffatomen; Kohlenwasserstoff mit bis zu 20 Kohlenstoffatomen mit Wasserstoff auf der Kohlenstoffkette substituiert durch F; Cl oder Br; aromatischer Kohlenwasserstoff mit bis zu 20 Kohlenstoffatomen ohne substituierte Gruppe oder aromatischer Kohlenwasserstoff mit bis zu 20 Kohlenstoffatomen mit Wasserstoff auf dem aromatischen Ring substituiert durch F; Cl; eine Amingruppe, Nitrilgruppe oder Alkoxy-Gruppe, sind.

12. Verfahren gemäß einem der Ansprüche 1 bis 11, wobei der Alkohol durch R'OH repräsentiert ist, wobei R' ein gesättigter linearer Kohlenwasserstoff mit bis zu 20 Kohlenstoffatomen; ein ungesättigter linearer Kohlenwasserstoff mit bis zu 20 Kohlenstoffatomen; ein Kohlenwasserstoff mit bis zu 20 Kohlenstoffatomen mit Verzweigungen; ein zyklischer Kohlenwasserstoff mit bis zu 20 Kohlenstoffatomen; ein aromatischer Kohlenwasserstoff mit bis zu 20 Kohlenstoffatomen, oder ein linearer Kohlenwasserstoff, der eine aromatische Gruppe enthält, ist.

13. Verfahren gemäß einem der Ansprüche 1 bis 12, wobei das Lösungsmittel repräsentiert wird durch die folgende Formel (III), (IV) oder(V):
**R**_{**3**}**―O―R**_{**4**} (III) 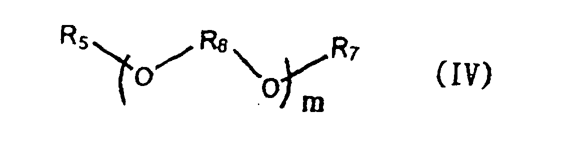 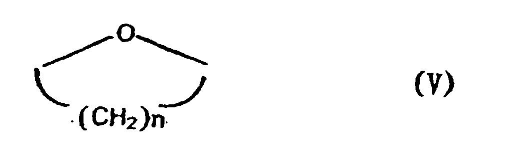
wobei R₃, R₄, R₅, R₆ und R₇ gesättigter aliphatischer Kohlenwasserstoff mit bis zu 10 Kohlenstoffatomen ohne Verzweigungen, verzweigter aliphatischer Kohlenwasserstoff mit bis zu 10 Kohlenstoffatomen; gesättigter zyklischer Kohlenwasserstoff mit bis zu 10 Kohlenstoffatomen; cykloaliphatischer Kohlenwasserstoff mit bis zu 10 Kohlenstoffatomen oder aromatischer aliphatischer Kohlenwasserstoff mit bis zu 10 Kohlenstoffatomen sind, m eine Ganzzahl von 1 bis 10 ist und n eine Ganzzahl von 2 bis 5 ist.

14. Verfahren gemäß einem der Ansprüche 1 bis 12, wobei das Lösungsmittel ein Acetat ist.

15. Verfahren gemäß einem der Ansprüche 1 bis 12, wobei das Lösungsmittel ein durch R'OH repräsentierter Alkohol ist, wobei R' ein gesättigter linearer Kohlenwasserstoff mit bis zu 20 Kohlenstoffatomen; ein ungesättigter linearer Kohlenwasserstoff mit bis zu 20 Kohlenstoffatomen; ein Kohlenwasserstoff mit bis zu 20 Kohlenstoffatomen mit Verzweigungen; ein zyklischer Kohlenwasserstoff mit bis zu 20 Kohlenstoffatomen; ein aromatischer Kohlenwasserstoff mit bis zu 20 Kohlenstoffatomen; oder ein linearer Kohlenwasserstoff, der eine aromatische Gruppe enthält, ist.

16. Verfahren gemäß einem der Ansprüche 1 bis 12, wobei das Lösungsmittel repräsentiert wird durch die folgende Formel (VI): 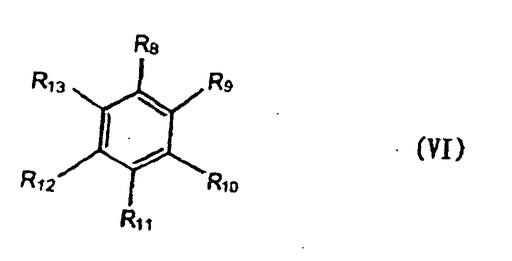 wobei R₈, R₉, R₁₀, R₁₁, R₁₂ und R₁₃ Wasserstoff, gesättigter aliphatischer C₁₋₄-Kohlenwasserstoff ohne Verzweigungen; verzweigter aliphatischer C₁₋₄-Kohlenwasserstoff; F; Cl oder eine C₁₋₃-Alkoxy-Gruppe sind.

17. Verfahren gemäß einem der Ansprüche 1 bis 16, wobei das Reaktionsprodukt 3-Hydroxyester und deren Derivate beinhaltet und repräsentiert wird durch die folgende Formel (VII) oder (VIII): 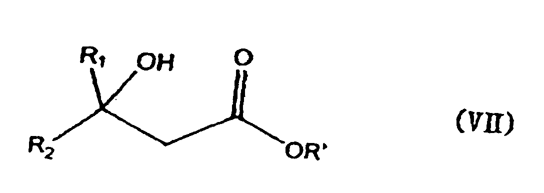 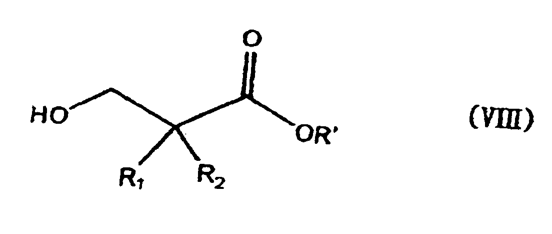 wobei R₁ und R₂ Wasserstoff, linearer aliphatischer Kohlenwasserstoff mit bis zu 20 Kohlenstoffatomen; verzweigter aliphatischer Kohlenwasserstoff mit bis zu 20 Kohlenstoffatomen; gesättigter cykloaliphatischer Kohlenwasserstoff mit bis zu 20 Kohlenstoffatomen; cykloaliphatischer Kohlenwasserstoff mit bis zu 20 Kohlenstoffatomen; aromatischer aliphatischer Kohlenwasserstoff mit bis zu 20 Kohlenstoffatomen; Kohlenwasserstoff mit bis zu 20 Kohlenstoffatomen mit Wasserstoff auf der Kohlenstoffkette substituiert durch F; Cl oder Br; aromatischer Kohlenwasserstoff mit bis zu 20 Kohlenstoffatomen ohne substituierte Gruppe oder aromatischer Kohlenwasserstoff mit bis zu 20 Kohlenstoffatomen mit Wasserstoff auf dem aromatischen Ring substituiert durch F; Cl; eine Amingruppe, Nitrilgruppe oder Alkoxy-Gruppe, sind, und R' ein gesättigter linearer Kohlenwasserstoff mit bis zu 20 Kohlenstoffatomen; ein ungesättigter linearer Kohlenwasserstoff mit bis zu 20 Kohlenstoffatomen; ein Kohlenwasserstoff mit bis zu 20 Kohlenstoffatomen mit Verzweigungen; ein zyklischer Kohlenwasserstoff mit bis zu 20 Kohlenstoffatomen; ein aromatischer Kohlenwasserstoff mit bis zu 20 Kohlenstoffatomen; oder ein linearer Kohlenwasserstoff, der eine aromatische Gruppe enthält, ist.

18. Verfahren gemäß einem der Ansprüche 1 bis 17, wobei das Katalysator-System des Schritts (c) Kupferchromat oder Pd/C ist.

19. Verfahren gemäß einem der Ansprüche 1 bis 18, wobei der Schritt (c) bei einem Wasserstoffdruck von 200 bis 3000 psig (1480 bis 20786 kPa absolut) durchgeführt wird.

20. Verfahren gemäß einem der Ansprüche 1 bis 19, wobei der Schritt (c) bei einer Temperatur von 50 bis 250°C durchgeführt wird.

## Revendications

1. Procédé de préparation d'alcane-1,3-diol par carbonylation d'un dérivé époxyde, qui comprend :
(a) la réaction d'un dérivé époxyde avec un alcool et du monoxyde de carbone dans un solvant à une température de 30 à 150°C et à une pression de 50 à 3 000 psig (446 à 20 786 kPa (absolu)) dans le système de catalyseur consistant en une quantité efficace d'un catalyseur au cobalt et une quantité efficace d'un promoteur choisi parmi l'imidazole, le pyrrole, la pyrazine, la pyrimidine, les dérivés de ceux-ci et les mélanges de ceux-ci pour convertir un 3-hydroxyester et les dérivés de celui-ci ;
(b) la séparation dudit produit de réaction et du solvant, du catalyseur et du promoteur ;
(c) la réaction dudit produit de réaction et du solvant avec de l'hydrogène à une température de 30 à 350°C et à une pression de 50 à 5 000 psig (446 à 34 576 kPa (absolu)) dans un système de catalyseur pour hydrogénation pour préparer un mélange de produit d'hydrogénation comprenant l'alcane-1,3-diol ; et
(d) la récupération de l'alcane-1,3-diol du mélange de produit d'hydrogénation.

2. Procédé selon la revendication 1, dans lequel ladite étape de séparation (b) est conduite sous distillation sous vide.

3. Procédé selon la revendication 1, dans lequel ladite étape de séparation (b) est conduite par extraction d'un 3-hydroxyester et de dérivés de celui-ci dans une couche d'eau en ajoutant de l'eau à une température de 100°C ou inférieure au mélange de production de réaction en présence de monoxyde de carbone à une pression de 20 à 3 000 psig (239 à 20 786 kPa (absolu)).

4. Procédé selon l'une quelconque des revendications 1 à 3, dans lequel lesdits catalyseur et promoteur sont recyclés vers l'étape (a) pour hydroestérification partielle ou totale.

5. Procédé selon l'une quelconque des revendications 1 à 4, dans lequel ledit catalyseur au cobalt est le Co₂(CO)₈.

6. Procédé selon l'une quelconque des revendications 1 à 5, dans lequel ledit catalyseur au cobalt et ledit promoteur sont employés à un rapport allant jusqu'à 1:100.

7. Procédé selon l'une quelconque des revendications 1 à 6, dans lequel ledit promoteur est un dérivé d'imidazole représenté par la formule (I) suivante : 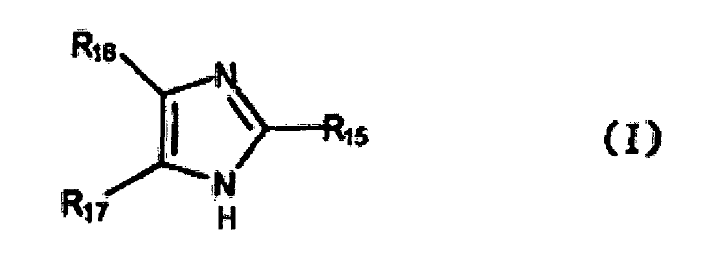 dans laquelle R₁₅, R₁₆ et R₁₇ représentent indépendamment un atome d'hydrogène ; un hydrocarbure aliphatique ramifié comportant jusqu'à 10 atomes de carbone ; un hydrocarbure aliphatique linéaire comportant jusqu'à 10 atomes de carbone ; un cyclohydrocarbure saturé comportant jusqu'à 10 atomes de carbone ; un hydrocarbure cycloaliphatique comportant jusqu'à 10 atomes de carbone ; un hydrocarbure aliphatique aromatique comportant jusqu'à 10 atomes de carbone ; F ; Cl ; un groupe alcoxy en C₁-C₃ ; OH ; un hydrocarbure aliphatique ramifié comportant jusqu'à 10 atomes de carbone contenant au moins un groupe OH; un hydrocarbure aliphatique linéaire comportant jusqu'à 10 atomes de carbone contenant au moins un groupe OH; un cyclohydrocarbure saturé comportant jusqu'à 10 atomes de carbone contenant au moins un groupe OH; un hydrocarbure cycloaliphatique comportant jusqu'à 10 atomes de carbone contenant au moins un groupe OH; ou un hydrocarbure aliphatique aromatique comportant jusqu'à 10 atomes de carbone contenant au moins un groupe OH.

8. Procédé selon la revendication 7, dans lequel ledit promoteur est un imidazole.

9. Procédé selon l'une quelconque des revendications 1 à 8, dans lequel ladite étape de réaction (a) est conduite à une température de 40 à 120°C.

10. Procédé selon l'une quelconque des revendications 1 à 9, dans lequel ladite étape de réaction (a) est conduite à une pression de 100 à 1 500 psig (790 à 10 444 KPa (absolu)).

11. Procédé selon l'une quelconque des revendications 1 à 10, dans lequel ledit dérivé époxyde est représenté par la formule (II) suivante : 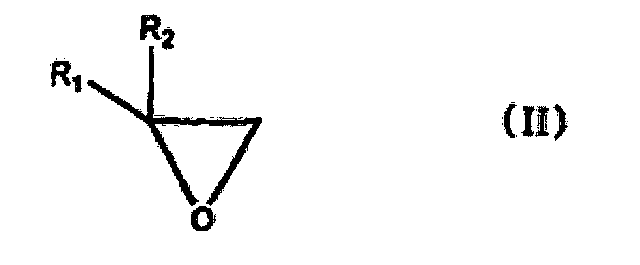 dans laquelle R₁ et R₂ représentent indépendamment un atome d'hydrogène ; un hydrocarbure aliphatique linéaire comportant jusqu'à 20 atomes de carbone ; un hydrocarbure cycloaliphatique ramifié comportant jusqu'à 20 atomes de carbone ; un hydrocarbure aliphatique saturé comportant jusqu'à 20 atomes de carbone ; un cyclohydrocarbure cycloaliphatique comportant jusqu'à 20 atomes de carbone ; un hydrocarbure aliphatique aromatique comportant jusqu'à 20 atomes de carbone ; un hydrocarbure comportant jusqu'à 20 atomes de carbone dans lequel des atomes d'hydrogène sur la chaîne de carbones sont substitués par F ou Cl ou Br ; un hydrocarbure aromatique comportant jusqu'à 20 atomes de carbone sans aucun groupe substitué ou un hydrocarbure aromatique comportant jusqu'à 20 atomes de carbone dans lequel des atomes d'hydrogène sur le noyau aromatique sont substitués par F, Cl, un groupe amine, un groupe nitrile ou un groupe alcoxy.

12. Procédé selon l'une quelconque des revendications 1 à 11, dans lequel ledit alcool est représenté par R'OH, dans lequel R' est un hydrocarbure linéaire saturé comportant jusqu'à 20 atomes de carbone, un hydrocarbure linéaire insaturé comportant jusqu'à 20 atomes de carbone ; un hydrocarbure comportant jusqu'à 20 atomes de carbone comportant des ramifications, un cyclohydrocarbure comportant jusqu'à 20 atomes de carbone, un hydrocarbure aromatique comportant jusqu'à 20 atomes de carbone ou un hydrocarbure linéaire comprenant un groupe aromatique.

13. Procédé selon l'une quelconque des revendications 1 à 12, dans lequel ledit solvant est représenté par la formule (III), (IV) ou (V) suivante :
**R**_{**3**}**―O―R**_{**4**} (III) 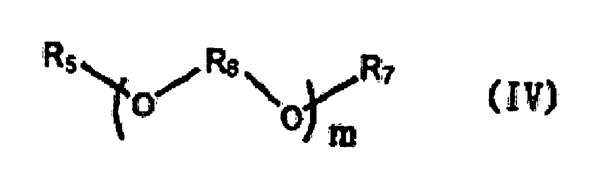 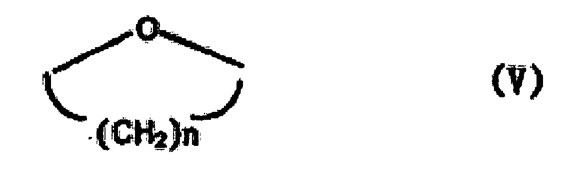
dans lesquelles R₃, R₄, R₅, R₆ et R₇ représentent un hydrocarbure aliphatique saturé comportant jusqu'à 10 atomes de carbone; n'ayant aucune ramification; un hydrocarbure aliphatique ramifié comportant jusqu'à 10 atomes de carbone ; un cyclohydrocarbure saturé comportant jusqu'à 10 atomes de carbone ; un hydrocarbure cycloaliphatique comportant jusqu'à 10 atomes de carbone ; ou un hydrocarbure aliphatique aromatique comportant jusqu'à 10 atomes de carbone ; m est un entier de 1 à 10 ; et n est un entier de 2 à 5.

14. Procédé selon l'une quelconque des revendications 1 à 12, dans lequel ledit solvant est un acétate.

15. Procédé selon l'une quelconque des revendications 1 à 12, dans lequel ledit solvant est un alcool représenté par R'OH, dans lequel R' représente un hydrocarbure linéaire saturé comportant jusqu'à 20 atomes de carbone, un hydrocarbure linéaire insaturé comportant jusqu'à 20 atomes de carbone, un hydrocarbure comportant jusqu'à 20 atomes de carbone comportant des ramifications, un cyclohydrocarbure comportant jusqu'à 20 atomes de carbone, un hydrocarbure aromatique comportant jusqu'à 20 atomes de carbone ou un hydrocarbure linéaire comprenant un groupe aromatique.

16. Procédé selon l'une quelconque des revendications 1 à 12, dans lequel ledit solvant est un composé aromatique substitué représenté par la formule (VI) suivante : 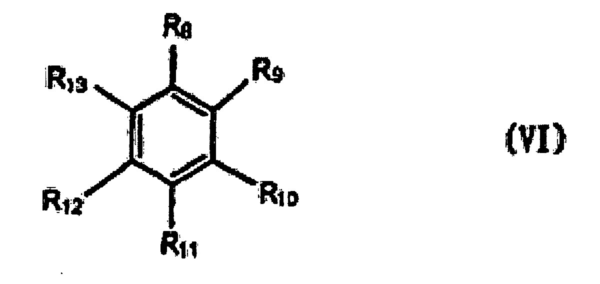 où R₈, R₉, R₁₀, R₁₁, R₁₂ et R₁₃ représentent un atome d'hydrogène ; un hydrocarbure aliphatique saturé en C₁-C₄ ne comportant aucune ramification ; un hydrocarbure aliphatique ramifié en C₁-C₄; F ; Cl ; ou un groupe alcoxy en C₁-C₃.

17. Procédé selon l'une quelconque des revendications 1 à 16, dans lequel ledit produit de réaction comprend un 3-hydroxyester et les dérivés de celui-ci représenté par l'une des formules (VII) ou (VIII) suivantes : 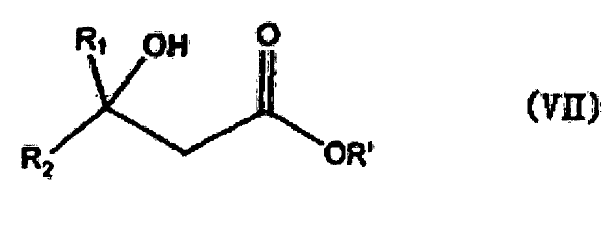 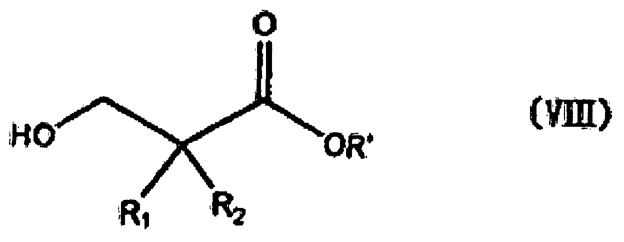 dans lesquelles R₁ et R₂ représentent un atome d'hydrogène ; un hydrocarbure aliphatique linéaire comportant jusqu'à 20 atomes de carbone, un hydrocarbure aliphatique ramifié comportant jusqu'à 20 atomes de carbone, un hydrocarbure cycloaliphatique saturé comportant jusqu'à 20 atomes de carbone ; un hydrocarbure cycloaliphatique comportant jusqu'à 20 atomes de carbone, un hydrocarbure aliphatique aromatique comportant jusqu'à 20 atomes de carbone ; un hydrocarbure comportant jusqu'à 20 atomes de carbone dans lequel des atomes d'hydrogène sur la chaîne de carbone sont substitués par F ou Cl ou Br ; un hydrocarbure aromatique comportant jusqu'à 20 atomes de carbone sans aucun groupe substitué ; ou un hydrocarbure aromatique comportant jusqu'à 20 atomes de carbone dans lequel des atomes d'hydrogène sur le noyau aromatique sont substitués par F, CI, un groupe amine, un groupe nitrile ou un groupe alcoxy, et R' représente un hydrocarbure linéaire saturé comportant jusqu'à 20 atomes de carbone, un hydrocarbure linéaire insaturé comportant jusqu'à 20 atomes de carbone, un hydrocarbure comportant jusqu'à 20 atomes de carbone présentant des ramifications, un cyclohydrocarbure comportant jusqu'à 20 atomes de carbone, un hydrocarbure aromatique comportant jusqu'à 20 atomes de carbone, ou un hydrocarbure linéaire comprenant un groupe aromatique.

18. Procédé selon l'une quelconque des revendications 1 à 17, dans lequel ledit système de catalyseur de ladite étape (c) est du chromate de cuivre ou du Pd/C.

19. Procédé selon l'une quelconque des revendications 1 à 18, dans lequel ladite étape (c) est conduite à la pression d'hydrogène de 200 à 3 000 psig (1 480 à 20 786 kPa (absolu)).

20. Procédé selon l'une quelconque des revendications 1 à 19, dans lequel ladite étape (c) est conduite à la température de 50 à 250°C.
